Europäisches Patentamt

(19) European Patent Office

Office européen des brevets

(11) Publication number: **0 240 074**
**A2**

(12) **EUROPEAN PATENT APPLICATION**

(21) Application number: **87200548.3**

(22) Date of filing: **25.03.87**

(51) Int. Cl.³: **G 01 L 5/04**

(30) Priority: **02.04.86 BE 2060957**

(43) Date of publication of application:
**07.10.87 Bulletin 87/41**

(84) Designated Contracting States:
**CH DE ES FR GB IT LI LU NL SE**

(71) Applicant: **Picanol N.V.**
**Polenlaan 3-7**
**B-8900 Ieper(BE)**

(72) Inventor: **Van Bogaert, Philippe**
**E. Cambierlaan 97**
**B-1030 Brussel(BE)**

(74) Representative: **Donné, Eddy**
**M.F.J.Bockstael Arenbergstraat 13**
**B-2000 Anvers(BE)**

(54) **Device for testing yarns.**

㊌ Device for testing yarns whereby this operation is automatically carried out on a large length of thread, characterized by the fact that it is mainly composed of the combination of a thread supply mechanism (1), of a thread guiding channel (2) which is mounted after the thread supply mechanism (1) of means for creating a fluid stream into the thread guiding channel (2) whereby the flow direction of the fluid is the same as the direction of the movement of the supplied thread, of a thread straining mechanism (3) which is mounted after the thread guiding channel (2) and of a thread break detector (4).

Fig.3

Device for testing yarns

-------------------------

The present invention concerns a device for testing yarns and mainly spun yarns which is designed in order to determine the maximum admissible stress of spun yarns, i.e. the stress which is not yet causing unacceptable wire breaks frequency, whereby the result obtained this way makes possible to adjust the weaving loom on the optimum way for weaving the tested yarns, or in other words, in order to be able to work with the maximum weaving speed, whereby a pre-determined average number of yarn breaks is not exceeded, related to a given number of shots or to the length of woven yarns.

Practically speaking, such an optimization results, for instance, for shuttle weaving looms, into an adaptation of the speed of the mechanical parts to the desired break limit which has been determined by the testing device for the yarns involved as well as to the weaving width. In the case of jet weaving looms, this optimization results into the application of a minimum permissible insertion time for a given yarn and for a given weaving width, with other words, the yarn stress occurring during the acceleration phase and/or the deceleration phase of the weft must be limited to the permissible stress. In the case of a gripper weaving loom, this optimization results into a driving speed caused by the grippers, which is adapted to the permissible stress and to an appropriate deceleration at the end of the weft insertion.

In order to achieve such an optimization, the yarn test has been carried out until now by means of precision devices whereby one or several weft ends of the yarn were cut-off in order to establish the stress - strain diagram up to the rupture limit. Such yarn tests have, however, the disadvantage that they are time-consuming and that in many cases, taking into account the small number of tests which are carried out, a misleading idea of the yarn characteristics is obtained for the tested yarn. Moreover, this already known device is fairly expensive.

Consequently, the present invention foresees a device for testing wires, whereby a quick quality control of the yarns can be carried out in the weaving mill, in such a way that a relatively quick forecast of the weaving output is possible. Moreover, the invention offers a device which permits to carry out continuous control of the yarn over long yarn lengths and also on full reels in such a way that the results obtained this way make it possible to determine with a very good reliability, the characteristics of the yarn. The quality difference within the same reel can also be merely indicated. Moreover, the device is a cheap one.

To this end, the device for testing yarns in accordance with the invention is mainly composed of the combination of a yarn supply mechanism, a wire guiding channel which is mounted after the yarn supply mechanism, of means to create a fluid stream in the thread guiding channel whereby the stream direction of the fluid is the same as the movement of the supplied thread; of a thread extension mechanism which is mounted after the thread guiding channel and of a thread break detector which is mounted after the thread supply mechanism in the path followed by the thread. According to a preferred embodiment, the device for testing yarns is also equipped with a stressmeter and with a thread guiding element in order to guide the tested yarn threads into a garbage bin.

In order to permit better understanding of the characteristics of the invention, a few preferred embodiments will be described hereafter as examples, without any limitative character and with reference to the figures in appendix, whereby:

figure 1 is illustrating two curves corresponding respectively to the stress applied by the weaving loom to the yarn and the breaking stress of the yarn itself.

figure 2 indicates the number of breaks per length unit of the yarns in function of the stress.

figure 3 schematically illustrates an embodiment of the device according to the invention.

figure 4 illustrates an example of the arrangement of a thread stressmeter for the device in accordance with the invention.

figures 5 to 7 give examples of the automatic insertion of the thread stress meter.

figures 8 to 11 give a few curves which can be determined while using the device in accordance with the invention.

figure 12 gives an alternative embodiment for the device in accordance with the invention.

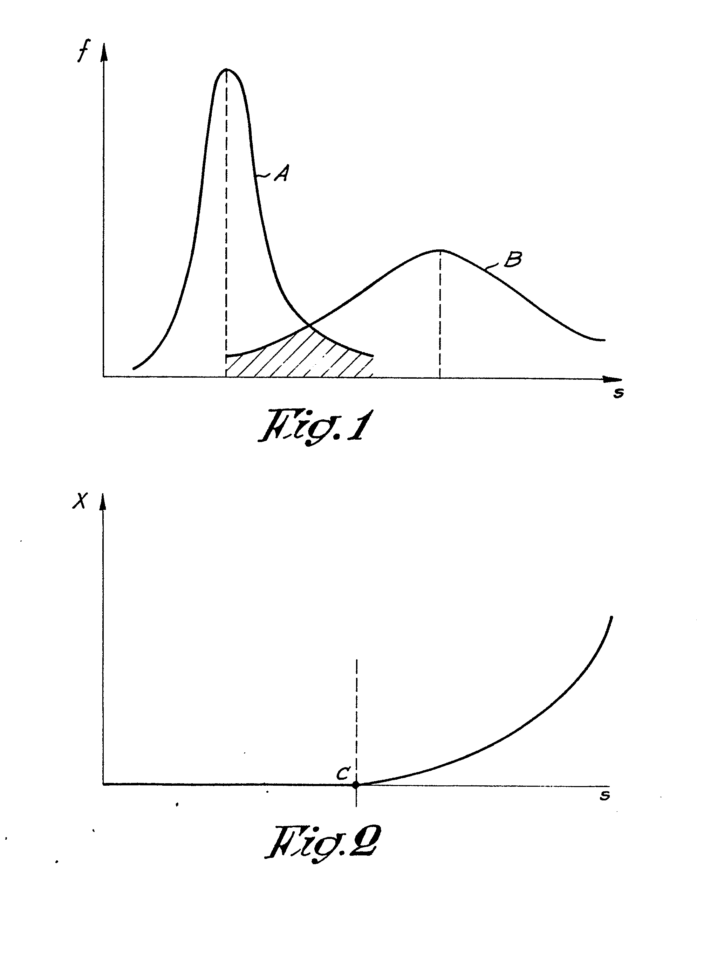

Figure 1 illustrates, on the one hand, a curve A which indicates the frequency f of the occurrence of specific stresses in the yarns, normally caused by the weaving loom, while curve B illustrates a so-called distribution curve for a given yarn. This distribution curve B indicates the density or the frequency f of the occurrence of breaks in the yarn in function of the stress s. Quite obviously, the cross-hatched surface of figure 1 is corresponding to a dangerous area whereby a large number of thread breaks can occur during the weaving process.

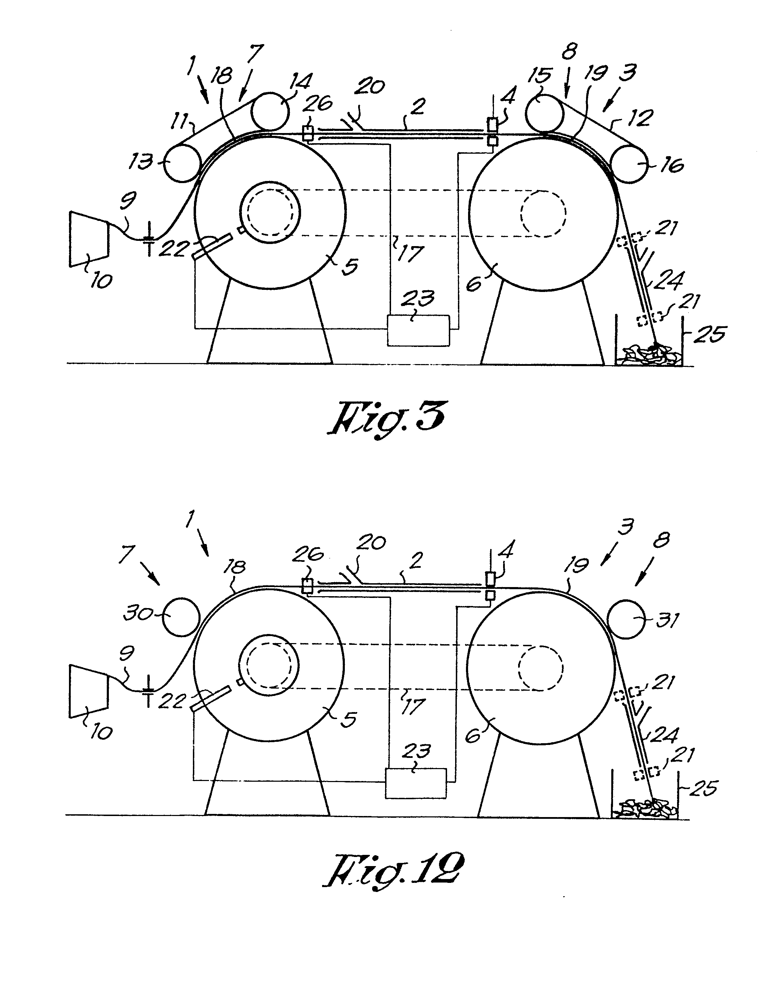

Figure 2 illustrates also the number of breaks X which is occurring or continuous stress for each kilometer of yarn in function of the stress s applied to the yarn. Quite obviously, no breaks will normally occur for the threads which are located on the left hand side of point C of figure 2, while, for threads located on the right hand side of point C, a critical condition is occurring, whereby the number of breaks per kilometer of yarn is rapidly increasing.

The theoretical description hereabove clearly indicates that it is of essential importance for a weaving mill to know with a good reliability the thread characteristics of the yarns to be woven, in such a way that the weaving speed can be adjusted in order to achieve the maximum weaving output. With other words, taking into account the known data about the thread characteristics, the weaving speed is increased in order that a determined number of thread breaks will not be exceeded. Practically speaking, this generally implies one break for 100.000 shots. The device described hereafter is characterized by the fact that the thread properties can be determined with a quite good reliability, as explained hereabove.

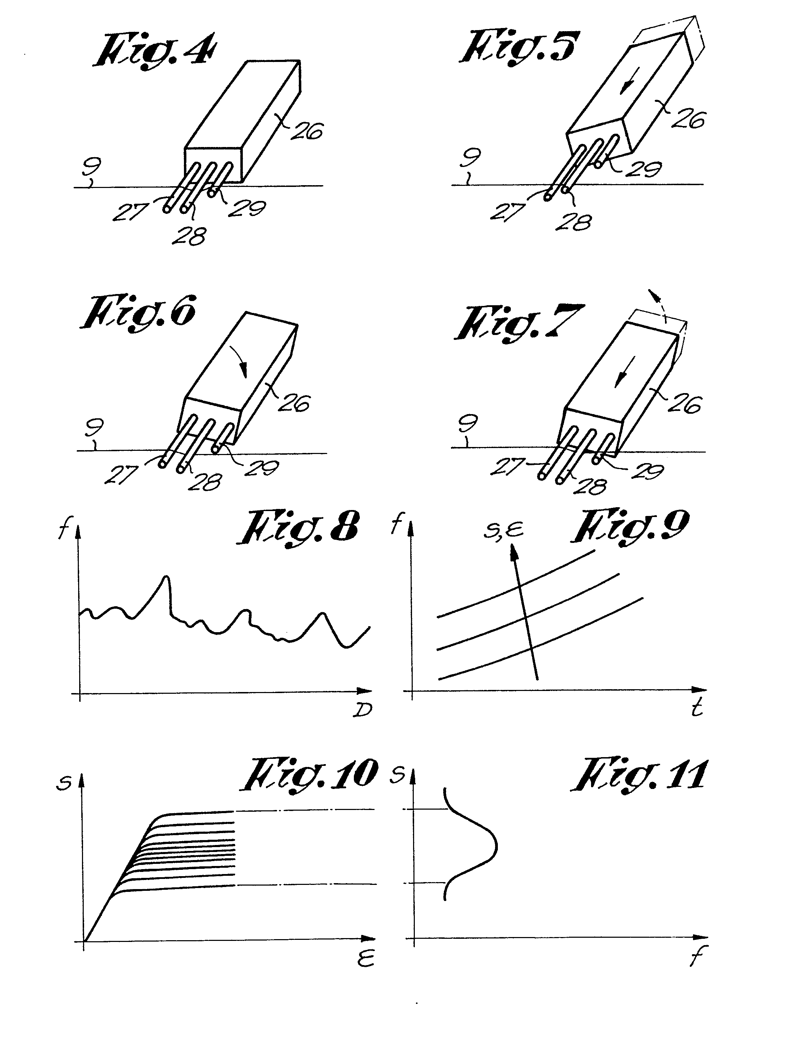

As illustrated on fig. 3, the device is mainly composed of a thread supply mechanism 1, of a thread guiding channel 2, which is mounted after the thread supply mechanism 1, of a thread straining mechanism 3 which is mounted after the thread guiding channel 2 and of a thread break detector 4, which is mounted, for instance, between the thread supply mechanism 1 and the thread straining mechanism 3. Moreover, means not illustrated on the figures are also foreseen in order to achieve a fluid stream in the thread guiding channel 2, which is moving from the thread supply mechanism 1 to the thread straining mechanism 3. The fluid is preferably composed of air.

In the preferable embodiment according to figure 3, the thread supply mechanism 1 as well as the thread straining mechanism 3 are mainly composed of two rigid wheels, respectively 5 and 6, whereon preferably elastic pressure elements 7 and 8 are acting, whereby a wire thread from a reel 10 mounted on an adequate manner can guided between the wheels 5 and 6 and respectively the pressure elements 7 and 8. The pressure elements 7 and 8 are mainly composed of moving belts 11 and 12 which are partially guided over the periphery of the wheels 5 and 6 by means of rolls 13 to 16.

The driving mechanism 3 for the thread is equipped with a transmission which is acting, for instance, on wheel 6. Wheel 5 of the thread supply mechanism is also coupled with this transmission or with the wheel 6, as illustrated on figure 3. In order to achieve a thread extension effect, the peripheric speed of wheel 6 is chosen somewhat higher than the peripheric speed of wheel 5. Quite obviously, this result can be achieved on quite various ways. In the embodiment according to figure 3, this result is obtained by means of a coupling 17 with a transmission ratio of 1:1 between wheels 5 and 6, whereby the diameter of wheel 6 is chosen larger than the diameter of wheel 5 in such a way that the thread 9 between both wheels 5 and 6 undergoes a well determined extension.

The thread guiding channel 2 is located in the continuation of, on the one hand, the contact surface 18 between wheel 5 and the pressure element 7 and, on the other hand, of the contact surface 19, between wheel 6 and the pressure element 8. Channel 2 is extending over all or part of the distance between the thread supply mechanism 1 and the thread straining mechanism 3.

Moreover, this thread guiding channel 2 is connected to aforesaid means in order to create a fluid stream whereby these means are composed, for instance, of a compressed air connection to a blow aperture 20, which is laterally discharging into the thread guiding channel 2.

The thread break detector 4 is preferably located near the thread straining mechanism 3 and shall be mounted, for instance, behind the thread guiding channel 2 or built in its wall. According to an alternative solution, the thread break detector may also be mounted on any arbitrary location after the thread straining mechanism 3 on the path of the yarn thread 9, as illustrated, for instance, in the figures 3 and 4, with the reference No. 21.

The thread break detector 4 is preferably of the opto-electronic, pneumatic or capacitive type.

The device in accordance with the invention is also equipped of a measuring device, in order to directly and/or indirectly measuring the quantity of threads already tested. To this end, at least one of the wheels 5 or 6 will be equipped for instance of a revolution counter, of a distance counter or of a speed meter 22. The revolution counter 22, as well as the thread break detector 4 may be coupled to a processing unit 23, for instance a micro-computer.

The device in accordance with the invention is equipped on a rear side of a thread guiding element 24, which is, for instance, of the pneumatic type and of a garbage bin 25.

As indicated on figure 3, the yarn testing device can be equipped, between the thread supply mechanism 1 and the thread straining mechanism 2 of a thread stressmeter 26 which is also coupled to the processing unit 23. The reason of this arrangement will be described hereafter. The thread stressmeter 26 can be made for instance of the device used and illustrated on figure 4, whereby the yarn thread 9 is guided between three contact elements 27, 28 and 29 and whereby, for instance, the central element 28 is detecting the stress in the yarn thread 9.

The thread stressmeter 26 described here by way of example can automatically come back after the rupture of the thread, while the yarn testing device gets a new thread afterwards. The thread stressmeter 26 is then automatically reset, whereby this result is obtained by means of a few translation and rotation movements, as illustrated on figures 5 to 7. As the contact elements 27, 28 and 29 don't have all three the same length, the yarn thread line can be replaced on the adequate manner between these elements. The thread stressmeter 26 is located preferably immediately after the thread supply mechanism 1, in such a way that the measurements carried out by the yarn testing device are influenced as little as possible.

The working of the yarn testing device is easily understood on figure 3 and mainly includes the fact that a yarn thread is guided from a reel 10 between wheel 5 and the pressure element 7 and is, afterwards brought into the thread guiding channel 2 and between wheel 6 and the pressure element 8, in order to finally arrive, along the thread guiding element 24, into a garbage bin 25. As a consequence of the different peripheric speeds of the thread supply mechanism 1 and of the thread straining mechanism 3, the yarn thread is quite obviously subjected to a specific stress between these both mechanisms, in function of the difference of the peripheric speeds. This results in a given number of thread breaks which can be counted by means of the thread break detector 4. If the speed of wheels 5 and 6 or the decoiling speed of the yarn is measured, the quantity of tested threads can be calculated by means of the processing unit 23 and consequently also the number of thread breaks per length unit, for instance per kilometer or per reel. In the case of the most simple test, the stress applied to the yarn thread is corresponding with the maximum stress which is occurring on a weaving loom for the normal weaving speed.

Quite obviously, the thread is reset into the device after each thread break, because the broken end is pulled through the thread guiding channel 2 and into the thread guiding element 24. These latter items should preferably be equipped of a suction aperture. According to a preferred embodiment, compressed air supply to the thread guiding element 24 shall be achieved only during a resetting operation.

The device should preferably work with a relatively low speed, in order that the influence of the reel on the stress in the yarn can be eliminated.

The device described hereabove is designed to carry out testing of thread lengths and preferably of a full reel for each batch of various yarns.

Although, as already explained, the device according to the invention can be applied in its most simple use for the determination of the average number of thread breaks per thread unit length under a specific stress, it is quite obvious that a large number of other measurements and operations can also be carried out if adequate adjustments are foreseen and with or without use of the stressmeter 26. A few of these applications are described hereafter.

If different stresses are applied to the yarn thread 9 during the test and if the number of breaks is determined in each case, while these data are processed by the processing unit 23, a diagram can be plotted, for instance with an ancillary device whereby the thread break frequency is illustrated in function of the applied stress. This way, we get a diagram as illustrated on figure 2.

According to another possibility, the thread break frequency is illustrated in function of the diameter D of the reel as shown on the curve given for instance on figure 8.

According to still another possibility, the influence of the duration of the stress on the thread break frequency is investigated. These tests can be carried out, for instance, by using different speeds for the device. If lower speeds are applied, each part of the thread will stay for a longer time between the wheels 5 and 6, whereby each thread part is also subjected to strain during a longer time.

According to an alternative solution, the adjustment of the duration is not achieved by speed regulation, but by adjustment of the distance between the thread supply mechanism 1 and the thread straining mechanism 3.

The influence of the timespan t where the stress is applied on the break frequency f can be illustrated by a curve for various stresses s (or for different strains) as shown on figure 9.

According to still another possibility the strain in the thread is modified during the test and the corresponding stresses in the thread are measured and processed. This way it is possible to obtain different curves which illustrate the stress in function of the strain on various locations in the test yarn thread 9, whereby these curves have the shape illustrated on figure 10. In these tests, the stress in the thread can be increased up to the thread rupture.

The density of these curves can be used to draw the break distribution curve according to figure 11 which is similar to the curve B of figure 1.

According to still another application of the device, measurements are carried out under constant strain. To this end, adjustment of the stress is foreseen.

Quite obviously, during the testing operations of the yarn thread 9, the strain or the stress in the yarn can be adjusted, regulated and controlled in quite various ways.

In order to modify the strain in the yarn thread 9, the difference of the peripheric speeds between wheels 5 and 6 can be increased or reduced. To this end, one of the wheels may be driven with a constant speed, while the other wheel is controlled by means of a feedback coupling. According to an alternative solution, a coupling 17 with an adjustable transmission ratio can be used like, for instance, a "variomatic" one. Quite obviously, other means already known can also be applied in order to achieve a controlled difference of the speeds of the thread supply mechanism and of the thread straining mechanism.

Further to the possibility of creating a speed difference between the thread supply mechanism 1, on the one hand, and the thread straining mechanism 3, on the other hand, the possibility of modifying the overall speed of the device should preferably also be foreseen. To this end, an adjustable device should be used in order to control, for instance, the peripheric speed of the wheels 5 and 6. This control can occur by means of a measuring device in order to determine the peripheric speed of the wheels 5 or 6 which can also be coupled to the processing unit 23.

The device, in accordance with the invention, can be put into practice by using a lot of various alternative designs. As illustrated, for instance, on figure 12, the pressure elements 7 and 8 may be composed of single pressure rolls 30 and 31, which are equipped with or without an elastic lining.

The present invention is by no means limited to the embodiments described by way of examples and in the figures in appendix, but these devices for testing yarns can be built with any shape and dimensions without departing from the scope of the invention.

Claims
------

1. Device for testing yarns, whereby this operation is automatically occurring over a large length of thread, characterized by the fact it is mainly composed of the combination of a thread supply mechanism (1); of a thread guiding channel (2) which is mounted after the thread supply mechanism (1); of means for creating a fluid stream into the thread guiding channel (2), whereby the flow direction of the fluid is the same as the movement direction of the supplied thread, of a thread straining mechanism (3) which is mounted after the thread guiding channel (2) and of a thread break detector (4) which is mounted after the thread supply mechanism (1) in the path which must be followed by the yarn thread (9).

2. Device for testing yarns according to claim 1, characterized by the fact that between the thread supply mechanism (1) and the thread straining mechanism (3) a thread stress meter (26) is mounted in the path to be followed by the yarn.

3. Device according to claim 1 or 2, characterized by the fact that the thread straining mechanism (3) is mainly composed of a driven wheel (6) and of a pressure element (8) which can cooperate with the periphery of wheel 6.

4. Device according to claims 1, 2 or 3, characterized by the fact that the thread supply mechanism (1) is composed of a driven wheel (5) and of a pressure element (7) which can cooperate with the periphery of wheel 5.

5. Device according to claims 3 or 4 characterized by the fact that wheel (6) of the thread straining mechanism (3) is coupled by a coupling (17) to the thread supply mechanism (1) in such a way that the peripheric speed of wheel (6) is larger than the speed of the movement imposed to the wire (9) by the thread supply mechanism (1).

6. Device according to claim 5, characterized by the fact that wheel (5) of the thread supply mechanism (1) is connected by means of a coupling (17) with a transmission ratio of 1/1 to the wheel (6) of the thread straining mechanism (3) and whereby the diameter of wheel (6) of the thread straining mechanism (3) is a little larger than the diameter of the wheel (6) of the thread supply mechanism (3).

7. Device according to claims or 4, characterized by the fact that means are foreseen in order to modify the speed difference between the speed of the yarn (9) on the thread supply mechanism (1) and the speed of the yarn (9) on the thread straining mechanism.

8. Device according to claim 7, characterized by the fact that the aforesaid means are composed of a coupling of the "variomatic" type between the thread supply mechanism (1) and the thread straining mechanism (3).

9. Device according to claim 7, characterized by the fact that aforesaid means imply that one of the wheels (5,6) is driven at a given speed, while the speed of the other wheel (5,6) is controlled by means of a feedback coupling.

10. Device according to one of the claims 3 or 4, characterized by the fact that at least one of the pressure elements (7-8) is made of a moving belt (11-12) which can cooperate with a part of the periphery of the corresponding wheel (5-6) of the thread supply mechanism (1) or of the thread straining mechanism (3) respectively.

11. Device according to one of the claims 3 to 10, characterized by the fact that a measurement device is foreseen for measuring the peripheric speed of wheel (5) of the thread supply mechanism (1) and/or of the wheel (6) of the thread straining mechanism (3).

12. Device according to one of the claims 3 to 11, characterized by the fact that an adjustable device is foreseen in order to modify the overall peripheric speed of aforesaid wheels (5-6).

13. Device according to one of the previous claims, characterized by the fact that a measuring device is foreseen for determining the quantity of tested yarn thread (9).

14. Device according to claim 13 characterized by the fact that the aforesaid measuring device is composed of a revolution meter (22) which is coupled with the thread break detector (4) to a processing unit.

15. Device according to one of the claims 2 to 12, characterized by the fact that a measuring device is foreseen in order to determine the quantity of tested yarn thread lines (9), whereby this measuring device, as well as the thread stress meter (26) and the thread break detector (4) are coupled to a processing unit (23).

16. Device according to one of the previous claims, characterized by the fact that the distance between the thread supply mechanism (1) and the thread straining mechanism (3) can be adjusted.

Fig.1

Fig.2

Fig.3

Fig.12

Fig.4

Fig.5

Fig.6

Fig.7

Fig.8

Fig.9

Fig.10

Fig.11